# EUROPÄISCHE PATENTSCHRIFT

(11) **EP 0 291 870 B1**
(45) Veröffentlichungstag und Bekanntmachung des Hinweises auf die Patenterteilung: **22.09.1993**
(21) Anmeldenummer: 88107690.5
(22) Anmeldetag: 13.05.1988
(51) Int. Cl.: C07C 69/30, C07C 67/03

(54) **Verfahren zur Herstellung von Triglyceriden**
Process for the production of triglycerides
Procédé de préparation de triglycérides

(30) Priorität: 20.05.1987 DE 3716950
(43) Veröffentlichungstag der Anmeldung: 23.11.1988
(73) Patentinhaber: Henkel Kommanditgesellschaft auf Aktien, 40191 Düsseldorf (DE)
(72) Erfinder: Peukert, Eberhard, D-4010 Hilden (DE); Rutzen, Horst, Dr., D-4018 Langenfeld (DE); Wollmann, Gerhard, Dr., D-4010 Hilden (DE)

(56) Entgegenhaltungen:
- EP-A- 0 150 794
- CH-A- 422 749

## Beschreibung

Die Erfindung betrifft ein Verfahren zur Herstellung von Triglyceriden von C₆-C₂₂-, insbesondere C₆-C₁₂-Fettsäuren durch Umsetzung von C₁-C₄-Alkylestern der Fettsäuren mit Glycerin in Gegenwart alkalischer Katalysatoren.

Die erfindungsgemäß herstellbaren Triglyceride sind handelsübliche Produkte mit einem breiten Anwendungsbereich. Von kurzkettigen (C₆-C₁₂) Fettsäuren abgeleitete Triglyceride, die z. B. unter der Bezeichnung Delios® in den Handel kommen, werden als Trägersubstanzen für Aromen und Essenzen, für die Oberflächenbehandlung von Trockenfrüchten und klebeempfindlichen Süßwaren, als Basisöl zur Herstellung von Trennmitteln für den Backwarensektor und dergleichen eingesetzt.

Bei der Herstellung von Methylestern durch Umesterung von Ölen, insbesondere von Kokosöl oder Palmkernöl, fallen bedingt durch die Natur des Ausgangsmaterials zwangsläufig große Mengen an sogenannten Vorlaufmethylestern, d. h. Methylestern des Kettenbereichs C₈-C₁₂ an. Die Menge dieser Vorlaufmethylester ist meist größer als der Bedarf, so daß ein Interesse an einem verfahren zur Herstellung von Triglyceriden aus den entsprechenden kurzkettigen Methylestergemischen auf direktem Wege ohne vorgeschaltete Umwandlung der Methylester zu den entsprechenden Fettsäuren besteht.

Es sind Verfahren zur Herstellung von Triglyceriden der eingangs genannten Art bekannt, wobei als alkalische Katalysatoren Natriummethylat und Natriumhydroxid verwendet werden. (EP-A-0 041 204) Die erhaltenen Triglyceride weisen jedoch eine dunkle Färbung auf und erfordern für Zwecke der Nahrungsmittelindustrie aufwendige Reinigungsoperationen. Weiterhin neigen die Reaktionssysteme zur Emulsionsbildung, und die als Nebenprodukte gebildeten Natriumsalze der Fettsäuren sind nur schwierig zu entfernen.

Die Erfindung ist auf ein Verfahren der eingangs genannten Art gerichtet, mit dem sich die vorgenannten Nachteile vermeiden lassen; insbesondere werden hellfarbige Trigiyceride erhalten.

Das Verfahren der Erfindung ist dadurch gekennzeichnet, daß man
a) den auf eine Temperatur von 150°C - 250°C erhitzten, getrockneten Fettsäurealkylestern ein Gemisch von trockenem Natriumcarbonat und Glycerin in einer Menge entsprechend einem molaren Verhältnis von Glycerin zu Fettsäurealkylester von 0,15 bis 0,30 und in einer Geschwindigkeit zudosiert, daß die Löslichkeit des Glycerins in dem Reaktionssystem nicht oder nicht wesentlich überschritten wird,
b) nach Zugabe des Glycerins dem siedenden Reaktionsgemisch weiteren Fettsäurealkylester in einer Menge von mindestens 10 %, bezogen auf in Stufe a) eingesetzten Fettsäurealkylester, zur Reaktion der nicht umgesetzten Hydroxylgruppen des Glycerins zusetzt,
c) die gesamte Reaktion unter leichtem Vakuum durchführt und gebildeten C₁-C₄-Alkohol ständig abdestilliert,
d) aus dem erhaltenen, überwiegend aus Fettsäuretriglycerid, Fettsäurealkylester und Natriumcarbonat bestehende Reaktionsgemisch den Fettsäurealkylester durch Destillation entfernt,
e) das Natriumcarbonat abtrennt und
f) das Fettsäuretriglycerid in üblicher Weise reinigt.

Als Berechnungsgrundlage für das Molekulargewicht der als Gemisch vorliegenden Fettsäurealkylester arbeitet man zweckmässigerweise mit demjenigen des C₉-Fettsäurealkylesters. Die Zugabe des Fettsäurealkylesters gemäß Stufe b) erfolgt auch zur Absenkung des Siedepunktes des Glycerid/Fettsäurealkylester-Gemisches.

Es ist wesentlich, daß man mit sorgfältig getrockneten Fettsäurealkylestern, Glycerin und Natriumcarbonat arbeitet, weil sogar bei Wassergehalten von nur 1 % im Reaktionsgemisch sich Seifen oder Emulsionen ausbilden.

Vorzugsweise setzt man als Fettsäurealkylester den Methylester ein.

Gemäß einer vorteilhaften Ausführungsform der Erfindung rektifiziert man den abdestillierten C₁-C₄-Alkohol, der stets etwas Fettsäurealkylester enthält, und führt den so gewonnenen Fettsäurealkylester in das Reaktionsgemisch zurück.

Es ist gemäß einer weiteren vorteilhaften Ausführungsform auch möglich, während der Reaktion das Gemisch von trockenem Natriumcarbonat in Glycerin direkt in die Rektifizierkolonne zuzugeben. Dabei findet schon in der Rektifizierkolonne eine Umsetzung zu Glyceriden statt, die zusammen mit nicht umgesetztem Fettsäurealkylester in das eigentliche Reaktionsgemisch zurückgeführt werden.

Gemäß einer weiteren vorteilhaften Ausführungsform der Erfindung kann man das Verfahren auch kontinuierlich durchführen. Diese Verfahrensvariante ist dadurch gekennzeichnet, daß man
a) eine eine Vielzahl von Böden aufweisende, beheizbare Reaktionskolonne mit aufgesetztem Rektifikationsaufsatz verwendet,
b) die Glycerin/Natriumcarbonat-Lösung nach Erhitzen auf die Reaktionstemperatur am oberen Teil der Reaktionskolonne aufgibt,
c) den Fettsäurealkylester in Form von gegebenenfalls überhitztem Dampf am mittleren Teil der Reaktionskolonne einspeist,
d) unterhalb des Einspeisungsortes des Fettsäurealkylesters in die Reaktionskolonne zur Reaktion des nicht umgesetzten Glycerins weiteren Fettsäurealkylester in Form von gegebenenfalls überhitzten Dampf einspeist,
e) an dem Rektifikationsaufsatz C₁-C₄-Alkohol und Fettsäurealkylester rektifiziert und den C₁-C₄-Alkohol am Kopf abzieht
   sowie
f) aus dem Sumpf der Reaktionskolonne Triglycerid/ Fettsäurealkylester/Natriumcarbonat-Gemisch entnimmt, an einem Sumpfverdampfer Fettsäurealkylester verdampft und als Dampfphase mindestens teilweise in die Kolonne oberhalb des Sumpfes zurückführt sowie erhaltenes Fettsäuretriglycerid vom Natriumcarbonat befreit und in üblicher Weise reinigt.

Die Erfindung wird anhand der folgenden Beschreibung näher erläutert, wobei auf die Zeichnungen Bezug genommen wird.

Es zeigen:
- Fig. 1: eine schematische Darstellung einer Anlage zur diskontinuierlichen Durchführung des Verfahrens der Erfindung und
- Fig. 2: eine schematische Darstellung des Reaktorteils einer Anlage für die kontinuierliche Durchführung.

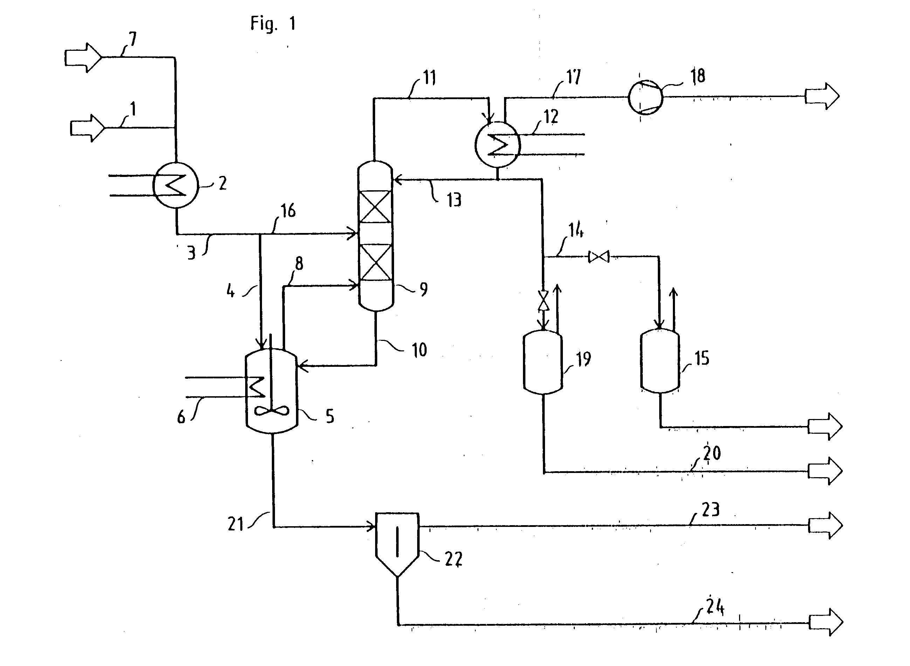

In der Anlage gemäß Fig. 1 wird Fettsäurealkylester über eine Leitung 1 einem Wärmeaustauscher 2 zugeführt und nach Erhitzen über Leitungen 3 und 4 in einen mit einem Rührwerk versehenen Reaktor 5 gegeben, der über eine Heizeinrichtung 6 beheizbar ist. Der Fettsäurealkylester wird im Reaktor bis zum Sieden erhitzt und, sofern dies nicht bereits vorher geschehen ist, getrocknet. Dem Reaktor wird weiterhin über eine Leitung 7 ein Gemisch von Natriumcarbonat und Glycerin zugeführt. Der sich im Verlauf der REaktion bildende C₁-C₄-Alkohol, der einen Teil des Fettsäurealkylesters enthält, wird über eine Leitung 8 am Kopf des Reaktors abgenommen und einer Rektifizierkolonne 9 zugeführt. In der Kolonne abgetrennter Fettsäurealkylester wird über eine Leitung 10 in den Reaktor 5 zurückgeführt. Der rektifizierte C₁-C₄-Alkohol wird am Kopf der Kolonne 9 abgenommen und über eine Leitung 11 einem Kondensator 12 zugeführt. Ein Teil des Alkohols kann über eine Leitung 13 als Rücklauf auf die Kolonne 9 zurückgegeben werden; der übrige Teil des Alkohols wird über eine Leitung 14 einem Vorratsbehälter 15 zur anderweitigen Verwendung zugeführt.

Die Rektifizierkolonne 9 kann über eine alternative Schaltung der Leitungen 7, 3 und 16 mit einem Gemisch von Natriumcarbonat und Glycerin beschickt werden, so daß in der Kolonne nicht nur eine Rektifizierung, sondern auch eine Umsetzung von Fettsäurealkylestern erfolgt. Dabei gebildetes Glycerid wird zusammen mit nicht umgesetzten Fettsäurealkylester in den Reaktor 5 zurückgegeben.

Die Anlage ist über eine Leitung 17 an eine Vakuumpumpe 18 angeschlossen und wird unter leichtem Vakuum betrieben. Dadurch kann eine niedrigere Reaktionstemperatur erreicht werden.

Nach Ablauf der Reaktion wird die Leitung 14 vom Vorratsbehälter 15 auf einen Vorratsbehälter 19 umgeschaltet.

Der nicht umgesetzte Ester wird bei erhöhter Temperatur und höherem Vakuum aus dem Reaktor 5 abdestilliert, im Kondensator 12 kondensiert und in dem Vorratsbehälter 19 zwischengelagert.

Über eine Leitung 20 wird der abdestillierte Fettsäurealkylester in der nächsten Charge in den Reaktor 5 zurückgeführt.

Nach der Destillation des Fettsäurealkylesters wird am Boden des Reaktors 5 ein Gemisch abgenommen, das im wesentlichen aus Triglycerid und Natriumcarbonat besteht, und über eine Leitung 21 einem Abscheider 22 zugeführt. Dort wird das Gemisch in seine Bestandteile aufgetrennt; das Triglycerid wird über eine Leitung 23 und der abgeschiedene Feststoff über eine Leitung 24 weggeführt.

Wesentliche Merkmale des Verfahrens der Erfindung bei diskontinuierlicher Arbeitsweise in einer Anlage gemäß Fig. 1 sind die folgenden:
1. Als Katalysator dient ein Gemisch von trockenem Natriumcarbonat in trockenem Glycerin. Natriumcarbonat ist im Glycerin in ausreichendem Maß löslich, nicht jedoch im Triglycerid, so daß es aus dem Reaktionsprodukt nach Abschluß der Reaktionsphase abfiltriert und erneut eingesetzt werden kann. Bei der Verwendung von Natriummethylat oder Natronlauge gemäß dem Stand der Technik sind hierzu Waschstufen vorzusehen, bei denen es leicht zu einer Emulsionsbildung kommt und ein erneuter Einsatz des Katalysators nicht möglich ist.
2. Die Steuerung des Reaktonsprozesses läßt sich dadurch erreichen, daß die Glycerinphase, in der der Katalysator gelöst ist, zu dem auf Reaktionstemperatur aufgeheizten Fettsäureester so zudosiert wird, daß entsprechend der Reaktionsgeschwindigkeit die Löslichkeitsgrenze des Glycerins im Ester nicht wesentlich überschritten wird. So wird praktisch ein Reaktionsablauf in homogener Phase erreicht und vermieden, daß durch Siedeverzüge oder zu starke Schaumbildung durch ausdampfendes Methanol der Prozeßablauf beeinträchtigt wird.
3. Die Hauptreaktion ist mit dem Ende der Zugabe von Glycerin und Natriumcarbonat weitgehend abgeschlossen. Die zeitlich länger als die Hauptreaktion dauernde Restreaktion erfordert ein Nachdosieren von Fettsäuremethylester zur Erhöhung bzw. zur Erhaltung des Fettsäureesterüberschusses. Die Zugabe dieses Esteranteils bereits bei Reaktionsbeginn wäre kein geeigneter Weg, denn in diesem Falle erreicht man keinen hinreichenden Umsatz, ausgedrückt durch die OH-Zahl von weniger als 5. In diesem Fall stagniert vielmehr die Reaktion in einem OH-Zahlbereich zwischen etwa 10 und 40 hängen. Das Reaktionsgemisch verbleibt auch in der Nachreaktionsphase im Siedezustand, d.h. bei konstantem Druck muß die Reaktionstemperatur so nachgeführt werden, daß stets ein Teil des Methylesters verdampft und im Rückfluß wieder zum Reaktor zurückgeführt wird, wobei jeweils Spuren an Reaktionsmethanol aus dem Reaktionsgemisch entfernt werden.
4. Nach der Restreaktion, wenn im Reaktor nur noch Triglyceride und überschüssiger Fettsäurealkylester vorliegt, wird der Überschußester bei einem möglichst niedrigen Druck abdestilliert, um die Temperatur im Reaktor niedrig halten zu können. Neben dem Katalysator ist im Reaktor dann nur noch das rohe Triglycerid vorhanden. Der abdestillierte Methylester kann mit der folgenden Charge vorgelegt werden.
5. Das mit Bildung des Triglycerids ausfallende Natriumcarbonat kann durch Filtration oder Zentrifugieren oder einen adäquaten Trennvorgang abgetrennt werden. Die Filtration erfolgt vorzugsweise bei 40-80°C. Der abfiltrierte Katalysator kann erneut eingesetzt werden.
6. Die Aufbereitung des Rohglycerids erfolgt anschließend nur noch über hier nicht gezeigte Destillationsstufen. Eine Bleichung und/oder Wäsche ist im allgemeinen nicht erforderlich.

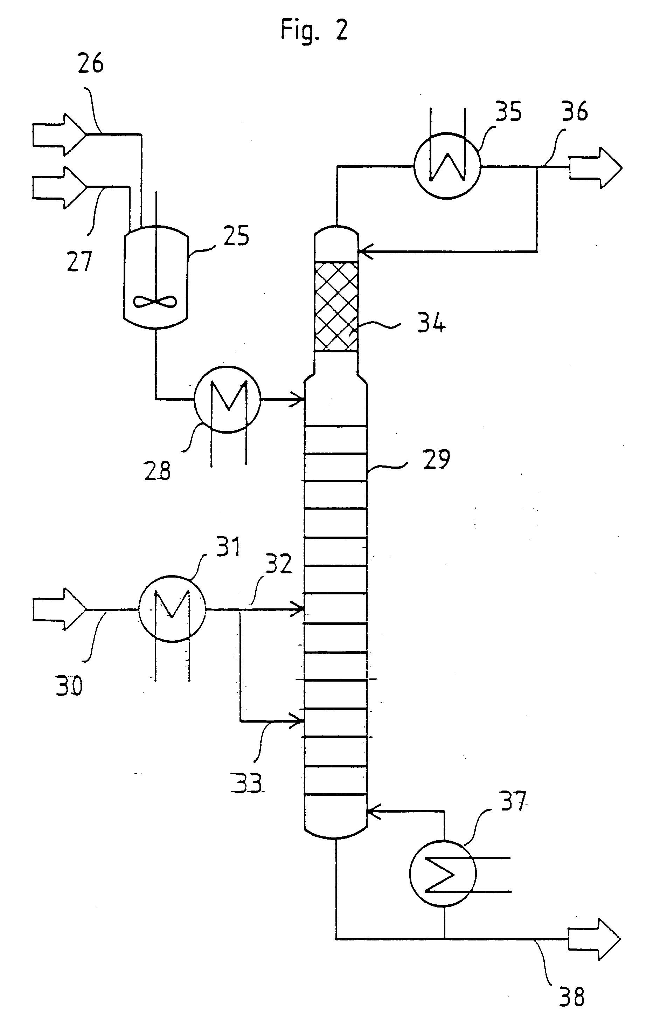

Die kontinuierliche Führung des Verfahrens der Erfindung wird anhand der Fig. 2 erläutert:

In einen Mischbehälter 25 wird Glycerin über eine Leitung 26 und Natriumcarbonat über eine Leitung 27 so eindosiert, daß sich das Natriumcarbonat im Glycerin löst. Die Lösung wird in einem Wärmeaustauscher 28 auf die Reaktionstemperatur gebracht und auf den obersten Boden einer Reaktionskolonne 29 in flüssiger Phase eingespeist.

Fettsäurealkylester wird über eine Leitung 30 einem Wärmeaustauscher 31 zugeführt, dort verdampft, gegenüber den Reaktionsbedingungen (Druck und Temperatur) überhitzt und in dieser Form als überhitzter Dampf über eine Leitung 32 und/oder 33 am mittleren Teil der Reaktionskolonne 29 eingespeist. Bei Durchströmung der Flüssigphase wird ein Teil des Esters kondensiert und reagiert, wobei als Reaktionsprodukt Methanol ensteht, welches verdampft und als überhitzter Dampf zusammen mit dem dampfförmigen Ester zum Kopf der Kolonne 29 strömt. Auf dem Weg des Dampfes zum Kopf der Kolonne nimmt demnach der Anteil an Methylester immer weiter ab, derjenige des Methanols nimmt zu.

Im Rektifikationsaufsatz 34 der Reaktionskolonne 29 wird der Rest Alkylester auf der Dampfphase abgetrennt; er gelangt als Flüssigphase wieder auf den obersten Boden der Reaktionskolonne, während der Reaktionsalkohol in einem Kondensator 35 kondensiert wird. Ein Teilstrom des Alkohols wird zum Rektifikationsaufsatz 34 zurückgeführt und der Hauptteil wird, entsprechend der Stoffbilanz der gesamte Reaktionsalkohol, über eine Leitung 36 aus dem System ausgeschleust.

Der für die Nachreaktion erforderliche zusätzliche Anteil an Fettsäurealkylester wird über die Leitung 33 unterhalb des ersten Aufgabeortes für den Fettsäurealkylester in die Reaktionskolonne eingespeist.

Im Sumpf der Reaktionskolonne 29 sammelt sich das gebildete Triglycerid mit dem entsprechenden Überschußanteil an Fettsäurealkylester. Es wird im Kreis über einen Sumpfverdampfer 37 geführt, wobei soviel an Methylester verdampft wird, daß die hydraulische Funktion des untersten Reaktionsbodens der Reaktionskolonne 29 gewährleistet ist. Aus dem Kreislaufstrom wird der Produktstrom des Glycerids über eine Leitung 38 abgetrennt. Von ihm muß in einer nachgeschalteten, nicht gezeigten Destillationsstufe der Restalkylester abdestilliert werden. Er kann über die Leitung 30 wieder der Reaktion zugeführt werden.

Druck und Temperatur in der Reaktionskolonne 29 werden gegenüber dem zur Reaktion zu bringenden Alkylester so eingestellt, daß dieser als überhitzter Dampf eingespeist werden kann.

Eine Besonderheit dieser Reaktionsführung liegt darin, daß im unteren Bereich der Reaktionskolonne ein Siedegleichgewicht zwischen Dampf- und Flüssigphase herrscht, während im oberen Teil der Kolonne der Stoffaustausch zwischen Flüssigkeits- und Dampfphase durch Absorption und Desorption bestimmt wird.

Das Verfahren der Erfindung wird im folgenden anhand von bevorzugten Ausführungsbeispielen näher erläutert.

### Beispiel 1

In einen 4 1-Glaskolben werden 2000 g Vorlaufmethylester C₆-C₁₂ vorgelegt.

Der Ester wurde bei 300 mbar und 150°C 25 Minuten getrocknet. 280 g Glycerin mit 13 g gelöstem Soda wurden innerhalb 2,5 h kontinuierlich zugeführt. Das Reaktionsprodukt Methanol verdampfte während der Glycerinzufuhr bei einer Sumpftemperatur von 140-155°C und einem Anlagendruck von 350-300 mbar. Der Dampf passierte eine ungeregelte Füllkörperkolonne mit unkontrolliertem Rücklauf. Der am Kopf der Kolonne austretende Dampf wurde kondensiert.

Nach einer Reaktionszeit von 3 h wurden nochmals 200 g Vorlaufmethylester innerhalb von 1/2 h in den Reaktor gegeben.

Unter Kochen am Rückfluß stieg zwischen 2,5 h und 4,5 h Reaktionszeit bei 300 mbar die Sumpftemperatur von 155 auf 180°C. Die Sumpftemperatur von 180°C und der Anlagendruck von 300 mbar wurden bis zum Beendigen der Reaktion nach 8 h Reaktionszeit beibehalten.

Insgesamt wurden 276 g Destillat aufgefangen. Nach 8,5 h wies eine Sumpfprobe eine OH-Zahl von 4,6 und eine SZ-Zahl von weniger als 0,1 auf. Die Farbzahlen des Sumpfes betrugen 6,0 gelb und 1,3 rot.

Der Sumpf wurde anschließend über PRIMISIL® (Filterhilfsmittel) filtriert; der ausgefallene Katalysator wurde auf diese Weise abgetrennt.

Bei einer Temperatur zwischen 145 und 205°C und einem Druck zwischen 150 und 60 mbar wurden zuerst 500 g Vorlaufmethylester aus der Blase abdestilliert. Anschließend wurden bei 170-247°C und einem Druck von 24-1 mbar 48,3 g dunkelgelber Farbvorlauf abdestilliert.

Die Triglyceride wurden bei 250°C und einem Druck von gleich oder weniger als 1 mbar überdestilliert. Man erhielt 91,2 % überdestillierte Triglyceride mit einer Farbzahl von 3 gelb und 0,5 rot, davon 85,5 % mit einer Farbzahl von 2 gelb und 0,5 rot.

Gegen Ende der Destillation dunkelte das Destillat wieder ein. Im Sumpf verblieben 16,5 g dunkelbrauner Rückstand.

### Beispiel 2

70 kg Vorlaufmethylester wurden in einen stickstoffgespülten 100 1-Reaktor vorgelegt und unter 300 mbar Vakuum auf 150°C aufgeheizt.

460 g Sodapulver wurden in 9,3 kg Glycerin gelöst; das Glycerin-Katalysator-Gemisch wurde innerhalb von 4 h in die siedende organische Phase dosiert.

Vom Beginn der Glycerinzufuhr an reagierten die Komponenten miteinander und es verdampfte ein Methanol-Ester-Gemisch. Der Dampf wurde rektifiziert; das Reaktionsprodukt Methanol wurde als Kopfkondensat aus dem System ausgeschleust. Nach der Glycerinzufuhr wurden nochmals 7 kg Vorlaufmethylester in das siedende Reaktionsgemisch innerhalb von 1,5 h eindosiert.

Damit das Sumpfgemisch weiterhin unter Kochen am Rückfluß siedete, wurde die Sumpftemperatur auf 180°C gesteigert. Der Druck blieb bei 300 mbar. Nach 11,25 h Reaktionszeit wies die Sumpfprobe eine OH-Zahl von 5,1 auf.

Anschließend wurde das gesamte Methanolkondensat aus der Kondensatvorlage entfernt.

Zum Abtreiben des nicht umgesetzten Esters wurde der Ester bei Sumpftemperaturen bis 183°C und einem absinkendem Vakuum bis 1,5 mbar unter Umgehung der Kolonne abdestilliert, kondensiert und separat aufgefangen. Nach der Esterrestdestillation wies die Sumpfprobe eine OH-Zahl von 3,0 auf.

## Patentansprüche

1. Verfahren zur Herstellung von Triglyceriden von C₆-C₂₂-, insbesondere C₆-C₁₂-Fettsäuren durch Umsetzung von C₁-C₄-Alkylestern der Fettsäuren mit Glycerin in Gegenwart alkalischer Katalysatoren, dadurch gekennzeichnet, daß man
a) den auf eine Temperatur von 150-250°C erhitzten, getrockneten Fettsäurealkylestern ein Gemisch von trockenem Natriumcarbonat und Glycerin in einer Menge entsprechend einem molaren Verhältnis von Glycerin zu Fettsäurealkylester von 0,15 bis 0,30 und in einer Geschwindigkeit zudosiert, daß die Löslichkeit des Glycerins in dem Reaktionssystem nicht oder nicht wesentlich überschritten wird,
b) nach Zugabe des Glycerins dem siedenden Reaktionsgemisch weiteren Fettsäurealkylester in einer Menge von mindestens 10 %, bezogen auf in Stufe a) eingesetzten Fettsäurealkylester, zur Reaktion der nicht umgesetzten Hydroxylgruppen des Glycerins zusetzt,
c) die gesamte Reaktion unter leichtem Vakuum durchführt und gebildeten C₁-C₄-Alkohol ständig abdestilliert,
d) aus dem erhaltenen, überwiegend aus Fettsäuretriglycerid, Fettsäurealkylester und Natriumcarbonat bestehende Reaktionsgemisch den Fettsäurealkylester durch Destillation entfernt,
e) das Natriumcarbonat abtrennt und
f) das Fettsäuretriglycerid in üblicher Weise reinigt.

2. Verfahren nach Anspruch 1 oder 2, dadurch gekennzeichnet, daß man als Fettsäurealkylester den Methylester einsetzt.

3. Verfahren nach Anspruch 1 oder 2, dadurch gekennzeichnet, daß man das abdestillierte C₁-C₄-Alkohol/Fettsäurealkylestergemisch rektifiziert und den Fettsäurealkylester in das Reaktionsgemisch zurückführt.

4. Verfahren nach einem der Ansprüche 1 bis 3, dadurch gekennzeichnet, daß man das Glycerin-Natriumcarbonat-Gemisch direkt in die Rektifizierkolonne einspeist.

5. Verfahren nach einem der Ansprüche 1 bis 4, dadurch gekennzeichnet, daß man zur kontinuierlichen Durchführung des Verfahrens
a) eine beheizbare Reaktionskolonne mit aufgesetztem Rektifikationsaufsatz verwendet, die eine Vielzahl von Böden aufweist,
b) das Glycerin/Natriumcarbonat-Gemisch nach Erhitzen auf die Reaktionstemperatur am oberen Teil der Reaktionskolonne aufgibt,
c) den Fettsäurealkylester in Form von gegebenenfalls überhitzem Dampf am mittleren Teil der Reaktionskolonne einspeist,
d) unterhalb des Einspeisungsortes des Fettsäurealkylesters in die Reaktionskolonne zur Reaktion des nicht umgesetzten Glycerins weiteren Fettsäurealkylester in Form von gegebenenfalls überhitzten Dampf einspeist,
e) an dem Rektifikationsaufsatz C₁-C₄-Alkohol und Fettsäurealkylester rektifiziert und den C₁-C₄-Alkohol am Kopf abzieht
sowie
f) aus dem Sumpf der Reaktionskolonne Triglycerid/Fettsäurealkylester/Natriumcarbonat-Gemisch entnimmt, an einem Sumpfverdampfer Fettsäurealkylester verdampft und als Dampfphase mindestens teilweise in die Kolonne oberhalb des Sumpfes zurückführt sowie erhaltenes Fettsäuretriglycerid vom Natriumcarbonat befreit und in üblicher Weise reinigt.

## Claims

1. A process for the production of triglycerides of C₆₋₂₂ fatty acids, more especially C₆₋₁₂ fatty acids, by reaction of C₁₋₄ alkyl esters of the fatty acids with glycerol in the presence of alkaline catalysts, characterized in that
a) a mixture of dry sodium carbonate and glycerol is added to the dried fatty acid alkyl esters heated to a temperature of 150°C to 250°C in a quantity corresponding to a molar ratio of glycerol to fatty acid alkyl ester of 0.15 to 0.30 and at such a rate that the solubility of the glycerol in the reaction system is exceeded only slightly, if at all,
b) after addition of the glycerol, more fatty acid alkyl ester is added to the boiling reaction mixture in a quantity of at least 10%, based on the fatty acid alkyl ester used in step a), to react the unreacted hydroxyl groups of the glycerol,
c) the entire reaction is carried out under a light vacuum and C₁₋₄ alcohol formed is continuously distilled off,
d) the fatty acid alkyl ester is removed by distillation from the reaction mixture obtained which consists predominantly of fatty acid triglyceride, fatty acid alkyl ester and sodium carbonate,
e) the sodium carbonate is separated off and
f) the fatty acid triglyceride is purified by standard methods.

2. A process as claimed in claim 1 or 2,characterized in that the methyl ester is used as the fatty acid alkyl ester.

3. A process as claimed in claim 1 or 2, characterized in that the C₁₋₄ alcohol/fatty acid alkyl ester mixture distilled off is rectified and the fatty acid alkyl ester is returned to the reaction mixture.

4. A process as claimed in any of claims 1 to 3, characterized in that the glycerol/sodium carbonate mixture is fed directly into the rectification column.

5. A process as claimed in any of claims 1 to 4, characterized in that, to carry out the process continuously,
a) a heatable, multiple-plate reaction column surmounted by a rectification column is used,
b) the glycerol/sodium carbonate mixture is introduced into the upper part of the reaction column after heating to the reaction temperature,
c) the fatty acid alkyl ester is introduced into the middle part of the reaction column in the form of optionally superheated vapour,
d) more fatty acid alkyl ester in the form of optionally superheated vapour is introduced below the point at which the fatty acid alkyl ester is fed into the reaction column in order to react the unreacted glycerol,
e) C₁₋₄ alcohol and fatty acid alkyl ester are rectified in the rectification column and the C₁₋₄ alcohol is distilled off overhead and
f) triglyceride/fatty acid alkyl ester/sodium carbonate mixture is removed from the sump of the reaction column, fatty acid alkyl ester is evaporated in a sump evaporator and returned at least partly to the column as vapour phase above the sump and fatty acid triglyceride obtained is freed from sodium carbonate and purified by standard methods.

## Revendications

1. Procédé de production de triglycérides d'acides gras en C₆ - C₂₂, en particulier de C₆ à C₁₂, par réaction d'esters d'alcoyle en C₁ à C₄ des acides gras avec du glycérol en présence de catalyseurs alcalins, caractérisé en ce que :
a) on ajoute par fractions à des esters d'alcoyle d'acides gras séchés, chauffés à une température de 150 à 250°C, un mélange de carbonate de sodium séché et de glycérol en quantité correspondant à un rapport molaire de glycérol à l'ester d'alcoyle d'acide gras de 0,15 à 0,30 et à une vitesse telle, que la solubilité du glycérol dans le système réactionnel n'est pas dépassée ou n'est pas fondamentalement dépassée.
b) après addition du glycérol au mélange réactionnel bouillant, on verse davantage d'ester d'alcoyle d'acide gras en une quantité d'au moins 10 % rapporté à l'ester d'alcoyle d'acide gras mis en oeuvre au stade a), en vue de la réaction des groupes hydroxyle du glycérol qui n'ont pas réagi.
c) on effectue la réaction totale sous léger vide et on sépare par distillation l'alcool en C₁ à C₄ formé, en permanence.
d) on élimine du mélange réactionnel formé principalement de triglycérides d'acide gras, d'esters d'alcoyle d'acide gras et de carbonate de sodium, les esters d'alcoyle d'acides gras par distillation.
e) on sépare le carbonate de sodium et,
f) on purifie le triglycéride d'acide gras d'une manière habituelle.

2. Procédé selon la revendication 1 ou 2, caractérisé en ce que l'on met en oeuvre comme ester d'alcoyle d'acide gras, l'ester méthylique

3. Procédé selon la revendication 1 ou 2, caractérisé en ce que l'on rectifie le mélange séparé par distillation d'alcool en C₁ - C₄/ester d'alcoyle d'acide gras et que l'on ramène l'ester d'alcoyle d'acide gras dans le mélange réactionnel.

4. Procédé selon l'une des revendications 1 à 3, caractérisé en ce que l'on introduit le mélange glycérol/carbonate de sodium directement dans la colonne de rectification.

5. Procédé selon l'une des revendications 1 à 4, caractérisé en ce que pour l'exécution en continu du procédé :
a - on utilise une colonne de réaction chauffable avec une soupape de rectification démontable, qui possède une multiplicité de plateaux.
b - on verse le mélange glycérol/carbonate de sodium après chauffage à la température de réaction à la partie supérieure de la colonne de réaction.
c - on introduit l'ester d'alcoyle d'acide gras sous forme de vapeur éventuellement surchauffée, à la partie moyenne de la colonne de réaction.
d - en dessous de l'emplacement d'introduction de l'ester d'alcoyle d'acide gras dans la colonne de réaction, en vue de la réaction du glycérol qui n'a pas réagi, davantage d'ester d'alcoyle d'acide gras est introduit sous forme de vapeur, éventuellement surchauffée.
e - sur la soupape de rectification, on a rectifié l'alcool en C₁ à C₄ et l'ester d'alcoyle d'acide gras et on a retiré l'alcool en C₁ à C₄ à la tête. ainsi que,
f - du bassin de pied de la colonne de réaction, on a enlevé le mélange triglycéride/ester d'alcoyle d'acide gras/carbonate de sodium, on a évaporé l'ester d'alcoyle d'acide gras sur un évaporateur du bassin de pied, on ramène dans la colonne audessus du bassin de pied au moins partiellement sous forme de phase vapeur ainsi qu'on débarrasse le triglycéride d'acide gras obtenu du carbonate de sodium et on purifie d'une manière habituelle.
